# EUROPEAN PATENT APPLICATION

(11) **EP 4 317 957 A1**
(43) Date of publication of application: **07.02.2024**
(21) Application number: 22382764.3
(22) Date of filing: 05.08.2022
(51) Int. Cl.: G01N 27/27, G01N 27/30, B29C 64/209, B29C 64/259, B33Y 10/00, B33Y 30/00, G01N 27/31, G01N 27/333, G01N 27/416, G01N 33/18, G01N 33/49

(54) **ADDITIVE MANUFACTURING OF SENSORS**

(71) Applicant: Fundació Eurecat, 08290 Cerdanyola del Vallès (ES)
(72) Inventor: MOYA LARA, Ana, 08302 MATARÓ (ES); ALIQUE GARCIA, Marc, 08302 MATARÓ (ES); BERENGUEL ALONSO, Miguel, 08302 MATARÓ (ES); FERNÁNDEZ ESPINAR, Daniel, 08812 BARCELONA (ES); DELGADO SIMAO, Claudia Custodia, 08302 MATARÓ (ES); LACHARMOISE, Paul Dominique, 08302 MATARÓ (ES)
(74) Representative: Igartua, Ismael

(57) **Abstract**

A method of additively manufacturing a sensor is provided. The method comprises providing an electrode, wherein the electrode comprises a conductive agent composition; and delivering a membrane agent composition onto the electrode to form a membrane. In addition, a system for additively manufacturing a sensor is provided. The system comprises a receiving region configured to receive the electrode; a membrane head for delivering the membrane agent composition onto the electrode through a nozzle; a container carrier attached to the membrane head; a driving arm configured to drive the membrane head at least over the receiving region; and a membrane head actuator configured to feed the nozzle with the membrane agent composition. Furthermore, a method of additively manufacturing a sensor assembly is provided.

## Description

The present disclosure relates to systems and methods for additively manufacturing a sensor. Furthermore, the present disclosure relates to systems and methods for additively manufacturing a sensor assembly.

### BACKGROUND

Sensors can be based, at least partially, on membranes (ion-selective membranes or ion-saturated membranes). Membranes may be formed onto an electrode of the sensor by drop casting a membrane agent composition. Drop casting is often a manual process in which a drop is cast on a substrate by an operator. Therefore, at least, reproducibility of those sensors is highly dependent on the skill or ability of the operator to perform a suitable drop cast of the membrane agent composition onto the electrode of the sensor at each performed drop cast. Particularly, drop casting may involve a labor-intensive process, which may limit scalability in manufacturing sensors.

In addition, drop casting allows very few parameters to be controlled. Typically, only a volume of the membrane agent composition delivered onto the electrode is controlled. By controlling only, the volume of the membrane agent composition delivered onto the electrode, there may be low reproducibility of the membrane agent composition delivered onto the electrode. Therefore, the formed membrane may be sensitive to a coffee ring effect and may not have a constant thickness. As a result, the manufactured sensor may have a poor performance, or may be even faulty.

Sensing devices for monitoring, e.g., water quality, blood, saliva, may comprise a sensor to determine a feature of interest e.g., redox, pH, free chlorine, conductivity, and temperature. Usually, only a feature of interest is measured at a time. If various features have to be measured, sensing devices have a stack of several sensors (e.g., redox sensor, pH sensor, free chlorine sensor, conductivity sensor, and temperature sensor).

For pH sensing, state-of-the-art glass electrodes are bulky, fragile, and expensive. Glass electrodes require calibration and cleaning of the electrode between each measurement. As a result, state-of-the-art glass electrodes are not suitable for portable applications.

In addition, free chlorine is often determined with optical methods to detect a color change of a fluid sample by adding N, N-diethyl-p-phenylenediamine (DPD). Detection of free chlorine may involve a reaction between an amine group of DPD and chlorine which produces a pink-colored compound. The color change is detected by an optical system. The optical system performs spectrum analysis of the fluid sample with DPD. However, this optical system is bulky and operated in laboratories. As a result, optical methods to determine free chlorine are complex, expensive, and are not suitable for portable applications nor performing continuous monitoring. At best, monitoring is performed at each retrieved fluid sample.

In summary, for detecting two or more features of interest, the sensing device may stack several sensors to form a sensor assembly. Therefore, the sensing device may include, e.g., glass electrodes for pH sensing; a light source, a cuvette, DPD, a photodetector, and circuitry for free chlorine sensing. As a result, the sensing device is bulky and may not be suitable for portable applications.

Examples of the present disclosure seek to at least partially reduce one or more of the aforementioned problems.

### SUMMARY

In one aspect, a system for additively manufacturing a sensor is provided. The system comprises a receiving region configured to receive an electrode; a membrane head for delivering a membrane agent composition onto the electrode through a nozzle. Particularly, the membrane head comprises a connecting port to receive an outlet end portion of a container to store the membrane agent composition. Furthermore, the system comprises a container carrier configured to hold the container through a carrier end portion of the container. The container carrier is attached to the membrane head. In addition, the system comprises a driving arm configured to drive the membrane head at least over the receiving region; and a membrane head actuator configured to feed the nozzle with the membrane agent composition, wherein the membrane head actuator is in fluid communication with the connecting port.

The configuration of the membrane head with the container by the action of the container carrier allows to efficiently feed the membrane agent composition from the outlet end portion of the container to the membrane head through the connecting port.

In this configuration, the outlet end portion of the container is connected to the connecting port of the membrane head. Therefore, the membrane agent composition is directly fed to the membrane head. There is no intermediate connector such as a feeding tube between the container and the membrane head. As a result, the compounds of the membrane agent composition cannot react with the feeding tube and inherently dissolve and / or deform, at least partially, the feeding tube. A deformed or misshaped feeding tube would inherently induce pressure changes which may alter reproducibility of the formed membrane. As there is no intermediate connector, pressure changes in the procedure may be reduced or even avoided. This way, reproducibility of the formed membrane may be improved. In addition, this configuration may avoid the substitution of a damaged feeding tube, and consequent interruption of manufacturing process.

Furthermore, as there is no feeding tube, the feeding tube has not to be filled for feeding the membrane head. According to this aspect, there is no feeding tube to couple the container with the membrane head. Therefore, the configuration of the membrane head with the container by the action of the container carrier may reduce the used amount of membrane agent composition. As a result, the manufacturing process may be more efficient and the cost of manufacturing a sensor may be reduced.

In some examples, the container carrier may comprise a carrier arm. In these examples, the carrier arm may comprise a hollow portion configured to receive at least the carrier end portion of the container. The carrier arm may engage at least the carrier end portion of the container. Therefore, the container carrier, e.g., by the hollow portion, may hold the container through the carrier end portion of the container. As a result, suitable placement of the container relative to the membrane head may be improved.

In some examples, the membrane head may have a generally elongated configuration such that a membrane head longitudinal axis is defined, and the container may be configured such that a container longitudinal axis is defined from the carrier end portion to the outlet end portion. In these examples, the container carrier may be configured to hold the container in such a way that an angle is defined between the head longitudinal axis and the container longitudinal axis. As a result, when the carrier arm engages at least the carrier end portion of the container, the angle may be maintained. Therefore, unfitting angles between the connecting port of the membrane head and the outlet end portion of the container may be avoided. Consequently, by the action of the container carrier, leaks of membrane agent composition may be avoided. Additionally, the connection between the container and the membrane head may be less vibration sensitive by the action of the container carrier, e.g., when the container carrier moves.

Because the membrane agent composition is delivered by a membrane head, parameters of the delivery are no longer limited to a volume of membrane agent composition; but there may be a plurality of parameters to control the suitable delivery of the membrane agent composition onto an electrode substrate. The plurality of parameters may comprise e.g., pressure; delivery speed; membrane agent composition feed rate; and distance between the nozzle outlet and the electrode substrate. By controlling an increased number of parameters, the delivered membrane agent composition may form a membrane with a constant thickness onto an electrode provided on the electrode substrate. As a result, the manufactured sensor may have an improved performance.

The above-mentioned parameters may be even more accurately controlled owing to synergistic effect of the direct connection between container and membrane head and the use of the membrane head. This may help to deliver membranes by additive manufacturing.

In a further aspect, a method of additively manufacturing a sensor is provided. The method comprises providing an electrode, wherein the electrode comprises a conductive agent composition; and delivering a membrane agent composition onto the electrode to form a membrane.

According to this aspect, providing an electrode may comprise an electrode substrate including an electrode (e.g., a first reference electrode; a second reference electrode; a first working electrode; a second working electrode; a third working electrode; and a counter electrode) or delivering, by additive manufacturing, a conductive agent composition onto an electrode substrate to form an electrode (e.g., a first reference electrode; a second reference electrode; a first working electrode; a second working electrode; a third working electrode; and a counter electrode).

Since the membrane of the sensor is additively manufactured, the membrane is no longer drop cast manually by an operator but by a system for additively manufacturing the sensor. Therefore, reproducibility of the manufactured sensor is not dependent on the skill or ability of the operator to perform a suitable drop cast of the membrane onto the electrode of the sensor but is dependent on controlled parameters (e.g., pressure; superficial tension; delivery speed; membrane agent composition feed rate; nozzle temperature; bed temperature; and distance between the nozzle outlet and the electrode substrate). As a result, the reproducibility of the membrane may be increased.

Furthermore, additively manufacturing the sensor may allow controlling more parameters than drop casting for delivering the membrane agent composition onto the electrode. Therefore, by increasing the controlled parameters, the formed membrane may have a substantially constant thickness onto the electrode. As a result, the performance of the additively manufactured sensor may be increased.

According to this aspect a sensor, including the membrane is additively manufactured. the scalability of manufacturing sensors comprising a membrane may be improved. Therefore, the cost and time of manufacturing sensors may be reduced.

In a further aspect, a method of additively manufacturing a sensor assembly is provided. The method comprises providing a sensor onto an electrode substrate, wherein the sensor is manufactured using a method of additive manufacturing according to any of examples disclosed herein; and providing a first reference electrode on the electrode substrate according to an example of the present disclosure. The method further comprises spray coating an IrOX agent composition onto a second working electrode provided on the electrode substrate, wherein the provided first reference electrode and the IrOX spray coated second working electrode forms a pH sensor.

In some examples, the additively manufactured sensor assembly onto the electrode substrate may comprise at least one of the following: a redox sensor; a pH sensor based on membranes; a pH sensor based on the IrOX agent composition; a chlorine sensor; a conductivity sensor; and a temperature sensor. The sensor assembly may comprise two or more sensors of the above-mentioned sensors. As a result, the sensor assembly may be suitable for portable applications because the sensor assembly comprises two or more sensors which are delivered onto the same electrode substrate.

In a further aspect, a method for manufacturing a microfluidic device is provided. The method comprises providing a microfluidic device including a structure; and connecting the microfluidic device with the sensor obtained by the methods according to the examples disclosed herein. In addition, the structure may comprise an inlet channel, an outlet channel, and a detection chamber in fluid communication with the inlet channel and the outlet channel. Furthermore, the microfluidic device may be connected with the sensor such that the detection chamber is fluidically connected to the electrode of the sensor. As a result, a fast point of care analysis device may be provided.

The term "additive manufacturing" may refer to automated processes in which successive layers are delivered on a manufacturing substrate. Automated processes may comprise screen- printing; 3D screen- printing; spray coating; binder jetting; material jetting; and material extrusion.

The term "structure" may be understood as a spatial pattern along 3 different directions (i.e., x, y, z) of a three-dimensional space which may be any suitable spatial arrangement such as, for example, a layer, a wafer, a cube, a cone, a cylinder, a disk, a hexagonal prism, a triangular prism, a pentagonal prism, a tetrahedron, an octahedron, a sphere and any combinations thereof.

The term "wt%" may be used to refer to a weight percentage of a first component (e.g., solid content) relative to the total weight of a second component (e.g., ink).

### BRIEF DESCRIPTION OF THE DRAWINGS

Non-limiting examples of the present disclosure will be described in the following, with reference to the appended drawings, in which:
Figure 1a schematically represents a block diagram of a method of additively manufacturing a sensor according to an example of the present disclosure;
Figure 1b schematically represents a block diagram of a method of additively manufacturing a sensor according to an example of the present disclosure;
Figure 2 schematically represents a block diagram of a method of additively manufacturing a sensor according to an example of the present disclosure;
Figure 3 schematically represents a block diagram of a conductive track manufacturing according to an example of the present disclosure;
Figure 4 schematically shows a block diagram of a spray coating of platinum agent composition according to an example of the present disclosure;
Figure 5 schematically shows a block diagram of a spray coating of IrOX agent composition according to an example of the present disclosure;
Figure 6 schematically shows a block diagram of a jetting of gold nanoparticle agent composition according to an example of the present disclosure;
Figure 7a schematically represents a sensor assembly comprising additively manufactured sensors according to an example of the present disclosure;
Figure 7b schematically represents the sensor assembly of figure 7a comprising conductive tracks according to an example of the present disclosure;
Figure 8 shows a cross-sectional view of a system for additively manufacturing the sensor according to an example of the present disclosure; and
Figure 9 shows a schematic view of a system for additively manufacturing the sensor according to an example of the present disclosure.

### DETAILED DESCRIPTION OF EXAMPLES

In these figures, the same reference signs have been used to designate matching elements.

The examples of methods disclosed herein are not constrained to a particular order.

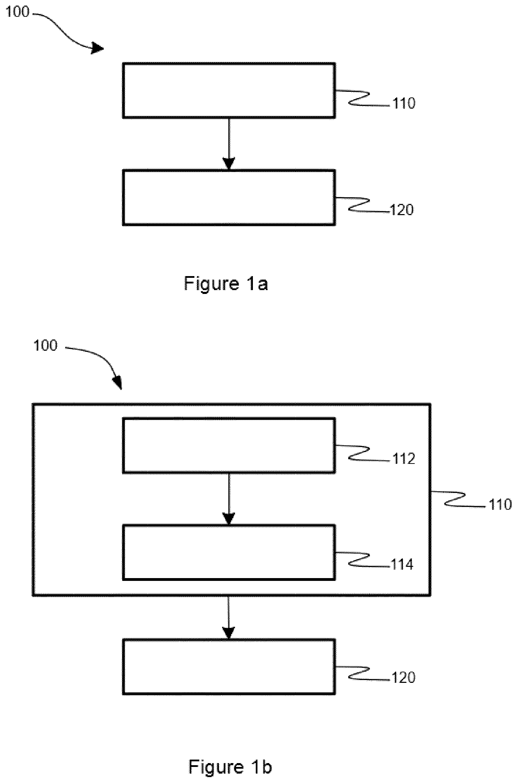

Figure 1a schematically shows a block diagram 100 of a method of additively manufacturing a sensor. A membrane of the sensor may be according to any of the examples herein disclosed. The method may be performed by a system for additively manufacturing a sensor according to any of the examples herein disclosed.

Throughout the description, the term "additive manufacturing" may be understood as automated processes in which successive layers are delivered onto a manufacturing substrate.

In some examples, the additive manufacturing of the present disclosure may be selected from screen- printing; 3D screen- printing; spray coating; binder jetting; material jetting (e.g., inkjet); and material extrusion.

In figure 1a, at block 110, an electrode is provided. Providing the electrode may comprise an electrode substrate including the electrode (e.g., a first reference electrode; a second reference electrode; a first working electrode; a second working electrode; a third working electrode; and a counter electrode). The electrode substrate may comprise one or more electrodes which may be provided onto or within the electrode substrate by additive manufacturing or another manufacturing process. For example, the provided electrode may be a screen-printed electrode.

Figure 1b schematically represents a block diagram of a method of additively manufacturing a sensor 100 according to an example of the present disclosure. In figure 1b, providing the electrode at block 110 may comprise delivering, by additive manufacturing, a conductive agent composition onto an electrode substrate to form the electrode, see block 112. In this example, providing the electrode at block 110 comprises additionally curing the conductive agent composition at block 114. Curing may be performed by heating the conductive agent composition, e.g., in an oven.

In summary, providing the electrode may comprise an electrode substrate including the electrode or delivering a conductive agent composition onto an electrode substrate to form the electrode.

The electrode, either provided by the electrode substrate including the electrode or provided by the delivered conductive agent composition onto the electrode substrate to form the electrode, comprises a conductive agent composition.

The conductive agent composition may comprise an electrically conductive compound selected from at least one of the following: e.g., silver; carbon; carbon allotropes; and copper. Particularly, the electrically conductive compound may have one to four valence electrons. In addition, the conductive agent composition may comprise a solid content between 10 and 90 wt% of the total weight of the ink; specifically between 20 and 80 wt%; and more specifically between 30 and 70wt%.

Referring to figures 1a and 1b, at block 120, a membrane agent composition is delivered onto the electrode to form a membrane. The membrane agent composition is delivered by additive manufacturing. The membrane agent composition may vary depending on a feature of the membrane to be formed. The feature may be related to a function of the membrane, e.g., ion-saturated, or ion-selective.

In some examples, the formed membrane may be an ion-saturated membrane. The ion-saturated membrane may be saturated with a specific ion. In these examples, the membrane agent composition which forms the ion-saturated membrane may comprise a polymer; a solvent; and a specific ion compound. Particularly, the polymer may be polyvinyl butyral (PVB); the solvent may be methanol; and the specific ion compound may be NaCI such that the ion-saturated membrane is saturated with Cl⁻ ions. In these examples, the feature of the membrane to be formed may comprised to be ion-saturated.

In some examples, the formed membrane may be an ion-selective membrane. The ion-selective membrane may be based on ionophores. lonophores are lipophilic complexing agents capable of reversibly binding a specific ion. For ion-selective membranes, the selectivity may depend on e.g., free energy of transfer of a specific ion from a fluid sample to the ion-selective membrane; a complex formation constant between the specific ion and the ionophore; and / or a concentration of the membrane compounds in the membrane. In these examples, the membrane agent composition which forms the ion-selective membrane may comprise a polymer, a solvent, an ionophore compound, an additive, and a plasticizer. Particularly, the polymer may be polyvinyl chloride (PVC), the solvent may be tetrahydrofuran (THF), the ionophore compound may be tri-n-dodecylamine (TDDA), the additive may be an anionic additive such as potassium tetrakis(4-chlorophenyl)borate (KTpCIPB). Because the TDDA is a hydrogen selective ionophore, the membrane, which may be formed from the membrane agent composition, may be selective to hydrogen ions (e.g., H⁺).

In some examples, the concentration of the anionic additive, i.e., KTpCIPB, may vary the ion-selectivity of the membrane which may be formed from the membrane agent composition.

In some of these examples, suitable plasticisers may comprise at least one of the following: bis(2-ethylhexyl)sebacate (DOS); 2-nitrophenyl octylether (NPOE); ortho-nitrophenyl octylether (o-NPOE); tris(2-ethylhexyl)phosphate; dibutyl sebacate; dioctyl sebacate; bis(2-ethylhexyl)adipate; bis(2-ethylhexyl)phthalate; dioctylphenylphosphonate; and mixtures thereof. The type of plasticizer used in the membrane may be chosen considering the composition of the polymer. In these examples, the plasticizer may be present in an amount of from 40 to 80 % by weight, specifically from 50 to 70 % by weight, more specifically about 66 % by weight. In these examples, the feature of the membrane to be formed may comprise to be ion-selective.

The membrane agent composition may be prepared using any suitable techniques for preparing membrane agent compositions, for example, by dissolving the compounds of the membrane agent composition in a solvent such as methanol or tetrahydrofuran. Other solvents or mixtures of solvents may be used. The membrane agent composition may be delivered by a system for additively manufacturing a sensor according to any of the examples herein disclosed. The formed membrane may be dried at ambient temperature.

For example, the ion-saturated membrane may be formed by the following membrane agent composition: PVB (61 wt%) dissolved in 5 mL of methanol. To this mixture may be added NaCl (39 wt%). The total weight of the membrane agent composition may be 645 g.

For example, the ion-selective membrane may be formed by the following membrane agent composition: PVC (32.4 wt%); o-NPOE (66 wt%); and KTpCIPB (0.6 wt%) dissolved in 5 mL of THF. To this mixture may be added TDDA (1 wt%). The total weight of the membrane agent composition may be 370 g.

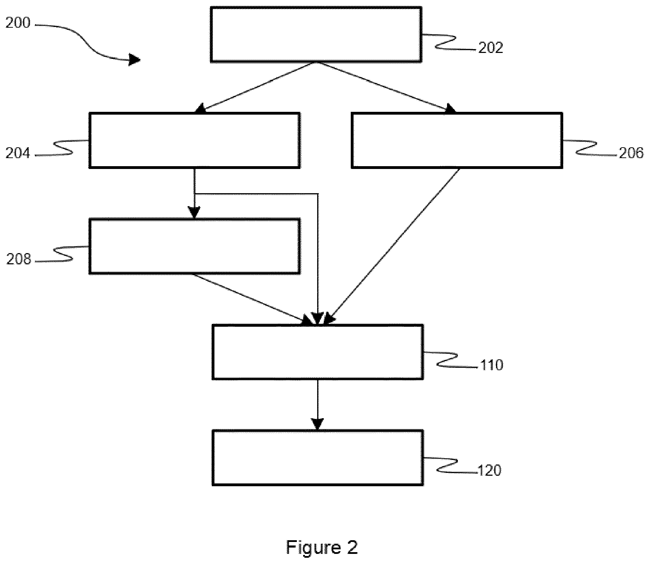

Figure 2 respectively shows a block diagram 200 of a method of additively manufacturing a sensor according to an example of the present disclosure.

In this example of figure 2, the electrode substrate providing the electrode is previously treated. At block 202, the electrode substrate may undergo a surface treatment prior to block 110. Surface treatments may be applied prior to block 110 for avoiding, at least in part, shrinkage of the electrode substrate when, e.g., the conductive agent composition is cured. In some examples, the electrode substrate may not undergo surface treatments prior to block 110.

During curing, the shape of e.g., the delivered conductive agent composition, or a further delivered agent composition may vary. The further delivered agent composition may be any agent composition which is further delivered onto the provided electrode. To avoid misshapes, the electrode substrate may be heated at a predefined temperature for a predefined time prior to delivering the conductive agent composition or the further agent composition. The electrode substrate may be heated at the predefined temperature depending on the highest curing temperature of the conductive agent composition or the further agent composition to be delivered onto the electrode substrate. The predefined time may be a time to which the conductive agent composition or the further agent composition with the highest curing temperature may be heated. For example, the delivered conductive agent composition at block 110 may be cured by heating the delivered conductive agent composition at 130°C for 30 minutes. In this example, there is a further agent composition that may be cured by heating the further agent composition at 140°C for 30 minutes. Therefore, in this example, the surface treatment may comprise heating the electrode substrate at 140°C for 30 minutes which are the highest curing temperature between the curing temperature of the conductive agent composition and the further agent composition. In this example, the predefined time may be 30 minutes because it is the time associated with the highest curing temperature.

Depending on the material of the electrode substrate, the surface treatment may be selected from at least one of the following: thermal treatment, oxygen plasma, and delivery of a dielectric agent composition onto the electrode substrate. Therefore, if the material of the electrode substrate changes, the surface treatment applied to the electrode substrate prior to block 110 may vary.

In some examples, the electrode substrate may be a polymer. In these examples, the polymer may undergo a thermal treatment at block 204 prior to block 110 (which may be the delivering the conductive agent composition onto an electrode substrate). The polymer may be selected from at least one of the following: polyethylene terephthalate PET; polyethylene naphthalate PEN; polyimide such as Kapton; polycarbonate PC; poly(methyl methacrylate) PMMA; thermoplastic polyurethane TPU; and paper.

In some examples, the electrode substrate may be a metal or a textile material. In these examples, the metal or the textile material may undergo a thermal treatment at block 204 prior to block 110, followed by delivering a dielectric agent composition onto at least a portion of the electrode substrate at block 208 prior to block 110 (which may be the delivering the conductive agent composition onto an electrode substrate). It is noted that the dielectric agent composition may be cured thereafter by heating or by exposure to electromagnetic radiation. The dielectric agent composition may be heated or exposed to electromagnetic radiation depending on its composition.

In some examples, the electrode substrate may be an oxide or a ceramic material. In these examples, the oxide or the ceramic material may undergo an oxygen plasma treatment at block 206 prior to block 110 (which may be the delivering the conductive agent composition onto an electrode substrate).

In summary, referring to figures 1a - b, and / or 2, the membrane may be additively deposited onto the electrode comprising the conductive agent composition.

In some examples, the conductive agent composition may comprise a first conductive ink. The first conductive ink may comprise at least one of silver, and silver chloride. When the provided electrode comprises the first conductive ink, the provided electrode may comprise a first reference electrode.

In some examples, the conductive agent composition may comprise a second conductive ink. The second conductive ink may comprise at least one of amorphous carbon, carbon black, and graphite. When the provided electrode comprises the second conductive ink, the provided electrode may comprise a counter electrode and / or a first working electrode and / or a second working electrode.

In some examples, the conductive agent composition may comprise a third conductive ink. The third conductive ink may comprise silver. When the provided electrode comprises the third conductive ink, the provided electrode may comprise a second reference electrode and / or a third working electrode made of silver.

In some of these examples and referring to the description of figures 1b and / or 2, delivering the conductive agent composition may comprise delivering at least one of the following: the first conductive ink with at least one of silver, and silver chloride; the second conductive ink with at least one of amorphous carbon, carbon black, and graphite, and the third conductive ink, such that the electrode is formed.

In addition, the delivered conductive agent composition onto the electrode substrate may be cured as aforementioned in the description of figure 1b at block 114.

The first conductive ink may be cured by heating the first conductive ink at 130°C for 30 minutes.

The second conductive ink may be cured by heating the second conductive ink at 130°C for 30 minutes.

The third conductive ink may be cured by heating the third conductive ink at 130°C for 30 minutes.

Therefore, the electrode substrate may comprise at least one of the following: the first reference electrode, the second reference electrode, the counter electrode, the first working electrode, the second working electrode, and the third working electrode.

In some examples, a surface of the electrode substrate may be fully covered by the formed electrode.

In some examples, the formed electrode may cover a portion of the surface of the electrode substrate.

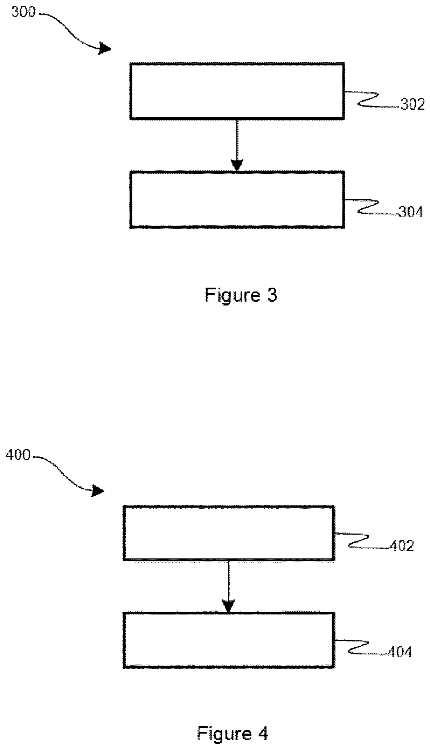

Figure 3 schematically represents a block diagram 300 of a conductive track manufacturing according to an example of the present disclosure. Features of figure 3 may be described in combination with the features of any of the examples shown in figures 1 - 2. The examples of methods shown in figure 1 - 2 may further comprise or not the features of the example of figure 3.

In the example of figure 3, the method of additively manufacturing a sensor comprises screen- printing a conductive track onto the electrode substrate at block 302. In some examples, screen- printing may be performed by a pneumatic screen- printer, specifically by a pneumatic flat screen-printer.

The conductive track may comprise an electrically conductive compound selected from at least one of the following: e.g., silver, carbon, carbon allotropes, and copper. As mentioned above, the electrically conductive compound may have one to four valence electrons. In this example of figure 3, the conductive track comprises silver.

At block 304, the screen-printed conductive track onto the electrode may be cured by heating the screen-printed conductive track, and exposing the screen-printed conductive track to an electromagnetic radiation. The screen-printed conductive track may be heated by exposure to electromagnetic radiation. The electromagnetic radiation may be provided by any type of electromagnetic radiation source that produces electromagnetic radiation in the wavelength range of 240 nm to 270 nm. In some examples, the electromagnetic radiation source can have a peak emission wavelength in this range. However, in other examples, the electromagnetic radiation source may have a peak emission wavelength outside this range. Even if the peak emission wavelength is outside the range, the electromagnetic radiation source can still produce enough radiation in the 240 nm to 270 nm range to heat the conductive track composition.

In some examples, the electromagnetic radiation source may include UV- light emitting diodes (LED), mercury lamps, etc. In some examples, the electromagnetic radiation source can include UV-LEDs that emit a center emission wavelength from 200 nm to 420 nm, specifically from 240 nm to 270 nm.

The rate at which an evaporable solvent evaporates from the screen-printed conductive tracks may be related to the intensity of the electromagnetic radiation applied. For instances, a lower intensity may involve a quick evaporation while a higher intensity may involve a longer evaporation. The intensity may be adjusted by selecting an electromagnetic radiation source with a desired power, or by adjusting a power density of the electromagnetic radiation source, and so on.

In summary, in the example of figure 3, the electrode is connected to an electrical contact through the conductive track.

Referring to the description of figures 1 - 3, the electrode substrate may comprise one or more electrodes, e.g., the first reference electrode, the second reference electrode, the counter electrode, the first working electrode, the second working electrode, and the third working electrode.

For obtaining a sensor, the membrane may be delivered onto the one or more electrodes, and / or further agent composition may be delivered onto the one or more electrodes. In both cases, the membrane and the further agent compositions may be delivered onto cured electrodes (i.e., provided electrodes which were cured beforehand, or formed electrodes which were cured after the conductive agent composition was delivered onto the electrode substrate).

Following figures and examples aim to explain how a sensor or a sensor assembly (e.g., redox sensor, pH sensor, chlorine sensor, conductivity sensor, and temperature sensor) may be obtained by applying, at least in part, the methods disclosed in the description of figures 1 - 3. Furthermore, two or more sensors may be obtained onto the same electrode substrate. The two or more sensors may be the same sensor (e.g., a first redox sensor and a second redox sensor) or different sensors (e.g., a redox sensor and a pH sensor).

Figure 4 schematically shows a block diagram 400 of a spray coating of platinum agent composition according to an example of the present disclosure. Features of figure 4 may be described in combination with the features of any of the examples shown in figure 1 - 3. The examples of methods shown in figure 1 - 3 may further comprise or not the features of the example of figure 4.

In figure 4, at block 402, a platinum agent composition is spray coated onto the first working electrode. In this example, the further agent composition may be the platinum agent composition.

In some examples, spray coating may be performed by using pressurized gas such as air at a flow rate of between 50 and 1000 µl/min; specifically between 100 and 800 µl/min; more specifically between 200 and 700 µl/min.

At block 404, the delivered platinum agent composition is cured by heating the platinum agent composition at 80°C for 10 minutes.

In this example of figure 4, the platinum agent composition may be a carbon supported platinum composition by a 3%wt of platinum.

It may be noted that curing parameters (e.g., heating temperature; and time in which the platinum agent composition is heated) may vary depending on the platinum agent composition. In some examples, the curing parameters may be different than the ones mentioned above.

Below, some examples of sensors are set forth. The sensors described below may be combined. A first sensor, a second sensor, and so on, may be additively manufactured onto the same electrode substrate. Additively manufacturing the first sensor onto the electrode substrate may not exclude additively manufacturing the second sensor onto the same electrode substrate. Therefore, the sensors below are not mutually exclusive.

### Redox sensor according to one example:

If the platinum agent composition is spray coated onto the first working electrode, and the membrane agent composition is delivered onto the first reference electrode to form the membrane, a redox sensor may be obtained. The obtained redox sensor may be a potentiometric sensor.

In these examples, the formed membrane onto the electrode is an ion-saturated membrane. The ion-saturated membrane may be manufactured according to the examples disclosed in the description of figure 1a and figure 1b. The ion-saturated membrane may be saturated in chloride ions so that the potential of the Ag / AgCI reference electrode (i.e., the first reference electrode) may remain stable at a predetermined reference electrode potential. In these examples, the ion-saturated membrane may be saturated in chloride ions so that the potential of the Ag / AgCI reference electrode (i.e., the first reference electrode) may remain stable at the predetermined reference electrode potential. Therefore, the ion-saturated membrane may keep substantially constant the concentration of chlorine ions in contact with the Ag / AgCI reference electrode.

### pH sensor according to one example:

A pH sensor may be obtained when the formed membrane onto the Ag / AgCI reference electrode (i.e., the first reference electrode) may be an ion-saturated membrane; and the formed membrane onto the first working electrode may be an ion-selective membrane which may be selective to hydrogen ions. The pH sensor may be a potentiometric sensor.

On the one hand, the ion-saturated membrane may be manufactured according to the examples disclosed in the description of figure 1a and figure 1b. The ion-saturated membrane may be saturated in chloride ions so that the potential of the Ag / AgCI reference electrode may remain stable at a predetermined reference electrode potential. In these examples, the ion-saturated membrane may be saturated in chloride ions so that the potential of the Ag / AgCI reference electrode may remain stable at a predetermined reference electrode potential. Therefore, the ion-saturated membrane may keep substantially constant the concentration of chlorine ions in contact with the Ag / AgCI reference electrode.

On the other hand, the ion-selective membrane may be manufactured according to the examples disclosed in the description of figure 1a and figure 1b. In these examples, the membrane, which may be formed from the membrane agent composition, may be selective to hydrogen ions (e.g., H⁺).

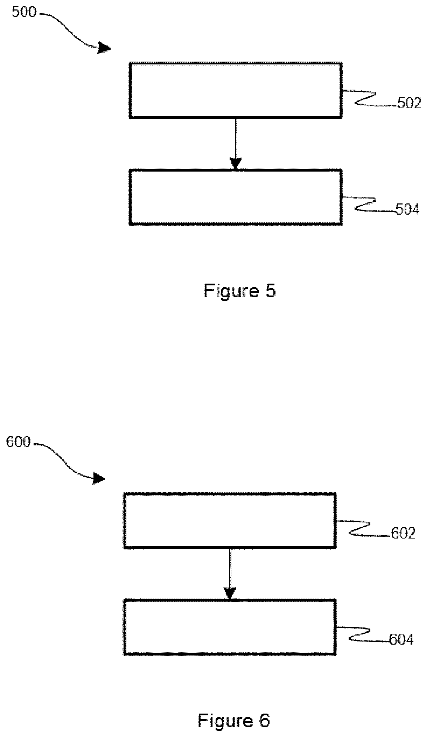

Figure 5 schematically shows a block diagram 500 of a spray coating of IrOX agent composition according to an example of the present disclosure.

In figure 5, at block 502, an IrOX agent composition is spray coated onto a second working electrode. In this example, the further agent composition may be the IrOX agent composition.

In some examples, spray coating may be performed by using pressurized gas such as air at a flow rate of between 50 and 1000 µl/min; specifically between 100 and 800 µl/min; more specifically between 200 and 700 µl/min.

At block 504, the delivered IrOX agent composition is cured by heating the IrOX agent composition at 80°C for 10 minutes.

The IrOX agent composition may comprise an iridium oxide compound, a solvent, and an ion-conducting polymer. The ion-conducting polymer may be an organic fluorinated polymer selected from homopolymers, copolymers, multicomponent polymers, or combinations thereof. In some examples, the ion-conducting polymer is a fluorinated polymer; particularly a fluorinated polymer having sulfonic acid sites; preferably a sulfonated tetrafluoroethylene based fluoropolymer or perfluorosulfonic acid (PFSA) polymer; more preferably is a tetrafluoroethylene-perfluoro-3,6-dioxa-4-methyl-7-octenesulfonic acid polymer or copolymer. In some of these examples, the fluorinated polymer may be compound of formula (I): 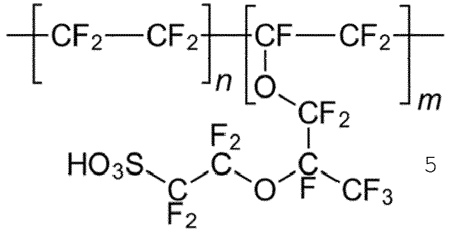 wherein n and m are integers which are above 1. In addition, the compound of formula (I) comprises an equivalent weight between 800 and 1400.

Furthermore, the iridium oxide compound may be IrOₓ, wherein x is equal to 2 or x is above to 2. In addition, the solvent may be a solvent which does not oxidize the IrOₓ compound and dilutes the fluorinated polymer for obtaining a viscosity and surface tension compatible with the delivery method (e.g., spray coating). For example, the viscosity and surface tension may be modified such that there may be a spray of the IrOX agent composition by using pressurized gas such as air at a predetermined flow rate. In some of these examples, the solvent may comprise propan-2-ol.

In the example of figure 5, a pH sensor is obtained when the IrOX agent composition is spray coated onto the second working electrode. In this example, the first reference electrode may be an Ag / AgCl reference electrode as described above.

It may be noted that in some examples, the IrOX agent composition may be used in a method of additively manufacturing a sensor assembly. The method comprises providing a sensor onto an electrode substrate, wherein the sensor is manufactured using a method of additively manufacturing a sensor according to an example of the present disclosure, providing a first reference electrode on the electrode substrate according to an example of the present disclosure; and spray coating the IrOX agent composition onto a second working electrode provided on the electrode substrate, wherein the provided first reference electrode and the IrOX spray coated second working electrode forms a pH sensor.

The pH sensor based on the IrOX agent composition may be suitable to obtain reliable measurement of pH in acidic or basic mediums. Particularly, the pH sensors based on the IrOX agent composition may have a pH operating range comprising acidic (e.g., pH < 4) or basic mediums (e.g., pH > 10.5).

The IrOX agent composition may allow an increased chemical stability of the pH sensor in the pH conditions of these mediums because of the low reaction of the IrOX agent composition with the pH conditions.

In some examples, the IrOX agent composition may be used in other system or methods which are not described in this disclosure.

### Chlorine sensor according to one example:

Figure 6 schematically shows a block diagram 600 of a jetting of gold nanoparticle agent composition according to an example of the present disclosure. Features of figure 6 may be described in combination with the features of any of the examples shown in figure 5. The examples of methods shown in figure 5 may further comprise or not the features of the example of figure 6.

In figure 6, at block 602, a gold nanoparticle agent composition may be jetted onto the second working electrode. In this example, the further agent composition may be the gold nanoparticle agent composition.

At block 604, the delivered gold nanoparticle agent composition may be cured by heating the gold nanoparticle agent composition at 140°C for 30 minutes.

The gold nanoparticle agent composition comprises a gold particle with a size between 1 nm and 100 nm

In figure 6, a chlorine sensor may be obtained when the gold nanoparticle agent composition may be jetted onto the second working electrode. In this example, the first reference electrode may be an Ag / AgCI reference electrode as described above. The chlorine sensor may output a signal representative of a presence of free chlorine. The jetted gold nanoparticle second working electrode may improve the stability over time (e.g., over 12 hours) of the chlorine sensor. Therefore, a calibration for compensating a change of response of the chlorine sensor over time may be avoided. As a result, the chlorine sensor may be used for successive measurements.

### Conductivity sensor according to one example:

By delivering the third conductive ink (as described above), a conductivity sensor may be obtained.

In some examples, the conductivity sensor may include two electrodes comprising a second reference electrode made of silver, and a third working electrode made of silver.

In some examples, the conductivity sensor may include four electrodes comprising a first pair of inner electrodes which include an inner second reference electrode and an inner third working electrode for potentiometric measurements (U); and a second pair of outer electrodes which include an outer second reference electrode and an outer third working electrode for amperometric measurements (I). In this configuration conductivity may be measured from the amperometric measurements (I) and the potentiometric measurements (U).

### Temperature sensor according to one example:

A temperature sensor may be obtained by delivering the conductive agent composition onto the electrode substrate. The temperature sensor may include a resistor formed of the conductive agent composition. In some examples, the resistor may have a positive temperature coefficient of resistance. The temperature sensor may comprise a first electrical contact at a first end of the resistor, a second electrical contact at a second end of the resistor, and a resistance measuring device connected to the first electrical contact and the second electrical contact to measure a resistance of the resistor (which may be correlated to the temperature).

The methods of additively manufacturing the sensor may result in manufacturing at least one of the following: the redox sensor; and the pH sensor based on at least the membrane.

The methods of additively manufacturing the sensor assembly according to an example of the present disclosure, may result in manufacturing at least one of the following: the pH sensor based on the IrOX agent composition, the chlorine sensor, the conductivity sensor, and the temperature sensor.

A combination of the above-described sensors may be obtained, for instance, in the same electrode substrate. Therefore, the combination of the above-described sensor may compose the sensor assembly. As a result, the sensor assembly may comprise two or more sensors of the above-mentioned sensors.

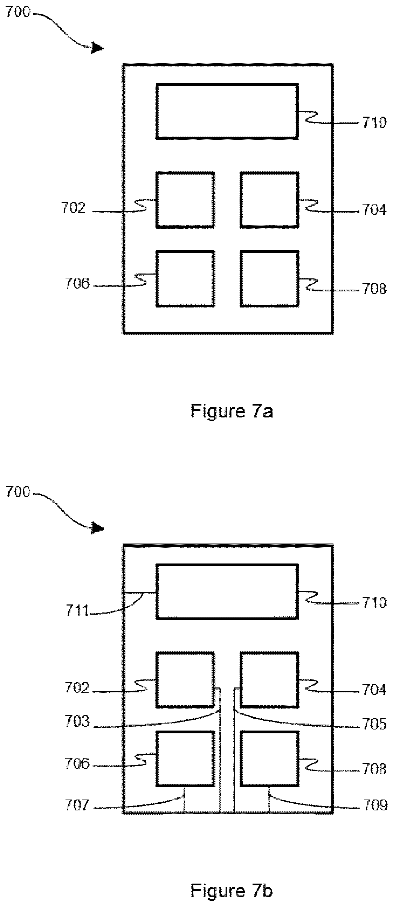

Figure 7a represents a sensor assembly 700 comprising additively manufactured sensors according to an example of the present disclosure.

In this example of figure 7a, the sensor assembly may be a stack of at least two of a redox sensor according to an example of the present disclosure 702, a pH sensor according to an example of the present disclosure 704, a chlorine sensor according to an example of the present disclosure 706, a conductivity sensor according to an example of the present disclosure 708, and a temperature sensor according to an example of the present disclosure 710.

It may be noted that the sensor assembly 700 may comprise any additively manufactured sensor according to the present disclosure (e.g., redox sensor; pH sensor; chlorine sensor, conductivity sensor, and temperature sensor). The sensor assembly at least comprises one sensor including a membrane delivered according to an example of the present disclosure.

Figure 7b schematically represents the sensor assembly 700 of figure 7a comprising conductive tracks according to an example of the present disclosure.

In this example of figure 7b, the sensor assembly further comprises conductive tracks. In figure 7b, the electrode of the redox sensor 702 is connected to a redox electrical contact through a redox conductive track 703, the electrode of the pH sensor 704 is connected to a pH electrical contact through a pH conductive track 705, the electrode of the chlorine sensor 706 is connected to a chlorine electrical contact through a chlorine conductive track 707, the electrode of the conductivity sensor 708 is connected to a conductivity electrical contact through a conductivity conductive track 709, and the temperature sensor 710 is connected to a temperature electrical contact through a temperature conductive track 711.

According to an aspect, a method for manufacturing a microfluidic device is disclosed. The method comprises providing a microfluidic device including a structure. Particularly, the structure comprises an inlet channel, an outlet channel, and a detection chamber in fluid communication with the inlet channel and the outlet channel. The method further comprises connecting the microfluidic device with the sensor obtained by the method according to an example of the present disclosure, or with the sensor assembly according to any of the examples of the present disclosure, such that the detection chamber is fluidically connected to the electrode of the sensor or to the electrode of the sensor assembly.

In some examples, the structure may comprise a first member structure and a second member structure. The first member structure may have a transition temperature Tg1, and the second member structure may have a transition temperature Tg2. The transition temperature Tg1 may be higher than the transition temperature Tg2.

In some examples, the first member structure and / or the second member structure may be a cyclic olefin copolymer (COC).

When the first member structure and the second member structure are bonded, fluidic channels may be milled through the second member structure and into the first member structure. The fluidic channels may comprise the inlet channel, the outlet channel, and the detection chamber. The detection chamber may be in fluid communication with the inlet channel and the outlet channel.

Connecting the microfluidic device with the sensor or the sensor assembly may be performed by fusing, at least partially, the second member structure with the electrode substrate of the sensor or of the sensor assembly. Because the second member structure may have a lower transition temperature, when a temperature t, Tg1 > t ≥ Tg2, and pressure is applied over the structure (comprising the first member structure and the second member structure) and the sensor or the sensor assembly, the second member structure may fuse, at least partially, with the electrode substrate. As a result, the microfluidic device may be obtained.

Therefore, a fluid sample may be provided in the inlet channel of the microfluidic device, wherein the provided fluid sample may be contacted with the electrodes or membranes. The provided fluid sample may reach the detection chamber by e.g., capillarity, gravity, pumping or the like. The contact between the fluid sample and the electrodes or membranes may generate a signal. The signal may be representative of: a presence or an absence of a target agent of the fluid sample, and / or an oxidation-reduction potential of the fluid sample, and / or a pH of the fluid sample, and / or a temperature of the fluid sample, and / or a conductivity of the fluid sample.

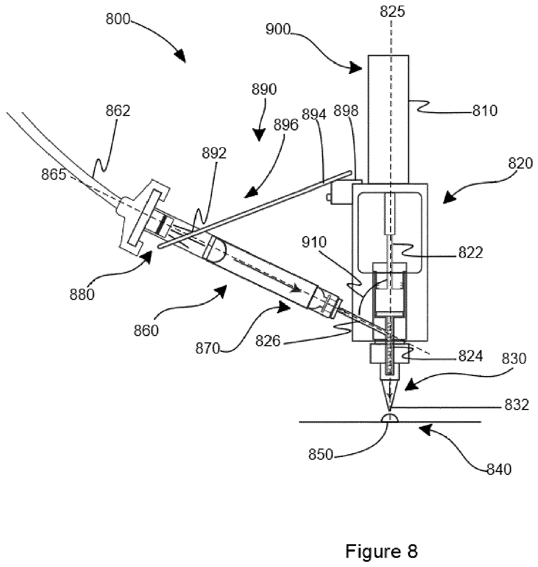

Figure 8 shows a cross-sectional view of a system 800 for additively manufacturing the sensor according to an example of the present disclosure.

The membrane head may have a generally elongated configuration such that a membrane head longitudinal axis 825 is defined. In some examples, the membrane head longitudinal axis 825 may be the same axis than a motor shaft axis.

In figure 8, the electric motor 810 is operatively connected with the membrane head actuator 822. The electric motor 810 may comprise a motor shaft defining a motor shaft axis. The motor shaft drives a membrane head actuator 822. The membrane head actuator 822 may be an endless screw. The membrane head actuator 822 may force the membrane agent composition to the nozzle 830 through a conduct 824. The conduct 824 is in fluid communication with the nozzle having a nozzle outlet 832. The membrane head actuator 822 may be in fluid communication with the connecting port 826 such that the membrane head actuator 822 may be configured to feed the nozzle 830. Therefore, the membrane agent composition may be delivered over a receiving region 840 to receive the electrode 850. The electrode 850 may be provided onto the electrode substrate (as described above) or provided by the delivered conductive agent composition onto the electrode substrate to form the electrode (as described above).

In figure 8, a driving arm 900 may be configured to drive the membrane head 820 at least over the receiving region 840, and the membrane head actuator 822 may be configured to feed the nozzle 830 with the membrane agent composition.

In addition, in figure 8, the membrane agent composition may be fed to the membrane head 820 through a connecting port 826 in fluid communication with the conduct 824 and a container 860.

Therefore, the system 800 for additively manufacturing the sensor comprises the receiving region 840 configured to receive an electrode 850, and the membrane head 820 for delivering a membrane agent composition onto the electrode 850 through a nozzle 830.

Furthermore, the container 860 may store the membrane agent composition to be fed to the membrane head 820. The container 860 may comprise an outlet end portion 870 and a carrier end portion 880. Therefore, a container longitudinal axis 865 may be defined from the outlet end portion 870 to the carrier end portion 880. The outlet end portion 870 may be directly connected to the connecting port 826.

In addition, the carrier end portion 880 of the container may be connected to a container actuator 862. The container actuator 862 may be configured to apply a pressure to the carrier end portion 880 to deliver the membrane agent composition through the outlet end portion. In some examples, the system 800 may comprise a controller configured to control a synchronous operation of the container actuator 862 and the membrane head actuator 822.

In figure 8, a container carrier 890 may have a distal portion 892 and a proximal portion 894. The container carrier 890 may be configured to hold the container through the carrier end portion 880 of the container. The container carrier 890 may be attached to the membrane head 820 by the proximal portion 894. The container carrier 890 may be configured to hold the container 860 in such a way that an angle 910 is defined between the membrane head longitudinal axis 825 and the container longitudinal axis 865.

In some examples, the container carrier 890 comprises a carrier arm 896, and the carrier arm 896 is connected to the membrane head 820 through a carrier adapter 898.

In some examples, the carrier arm 896 may be hingedly connected to the carrier adapter 898. This way, the carrier arm 896 may adopt different position relative to the membrane head 820. This may help to connect containers having different shapes or sizes.

In some examples, the carrier arm 896 comprises a hollow portion configured to receive at least the carrier end portion 880 of the container. The hollow portion may comprise an aperture which may surround the carrier end portion 880 of the container when the carrier end portion 880 of the container engages the aperture.

Referring back to the container 860, in some examples, the container 860 may be a syringe. The syringe may comprise a barrel having a hollow inner portion; a plunger to be placed in the hollow inner portion, the plunger being movable along a longitudinal axis of the barrel; wherein the plunger is operatively connected to the container actuator 862; and a syringe outlet in fluid communication with the hollow inner portion. The longitudinal axis of the barrel may be the container longitudinal axis 865.

The container actuator 862 may be configured to drive the plunger for delivering the membrane agent composition from the syringe outlet to the connecting port 826.

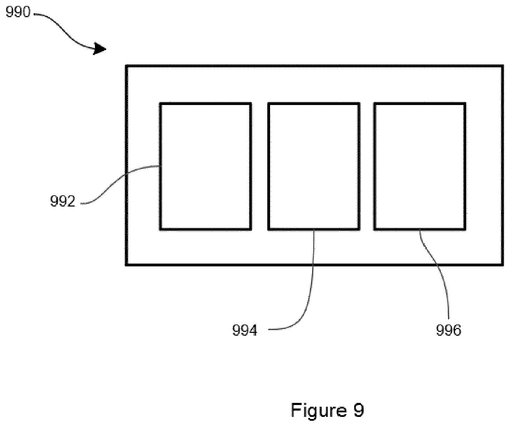

Figure 9 shows a schematic view of a system 990 for additively manufacturing the sensor according to an example of the present disclosure.

The system 990 may comprise a first delivery unit 992 configured to deliver the conductive agent composition onto the electrode substrate to form the electrode. The system 990 may comprise a curing unit 994 to cure the delivered conductive agent composition; and a second delivery unit 996. The second delivery unit 996 is configured to deliver, at least, the membrane agent composition onto the electrode to form the membrane.

In some examples, the second delivery unit 996 may comprise the receiving region 840 configured to receive the electrode 850, and the membrane head 820 for delivering the membrane agent composition onto the electrode 850 through the nozzle 830. The membrane head 820 may include the connecting port 826 to receive the outlet end portion 870 of the container 860 to store the membrane agent composition. In addition, the second delivery unit may comprise the container carrier 890 configured to hold the container 860 through a carrier end portion 880 of the container. The container carrier 890 may be attached to the membrane head 820. The system 990 may comprise the driving arm 900 configured to drive the membrane head 920 at least over the receiving region 840; and the membrane head actuator 822 configured to feed the nozzle 830 with the membrane agent composition, wherein the membrane head actuator 822 is in fluid communication with the connecting port. So, the second delivery unit 996 may comprise a system according to the example of figure 8.

In some examples, the system 990 may comprise an illuminating device to emit an electromagnetic radiation with a wavelength comprised between 100 nm and 400 nm on the electrode substrate. This wavelength may be useful for curing some curable agent compositions.

According to an aspect an IrOX agent composition is disclosed. The IrOX agent composition comprising: an iridium oxide compound, a solvent; and a compound of formula (I): 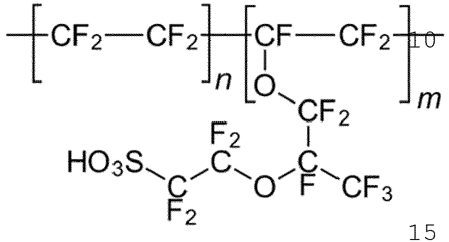 wherein n and m are integers which are above 1.

Although this IrOX agent composition has been described in relation with examples of systems or methods for additively manufacturing a sensor as disclosed herein, the IrOX agent composition can be used in other examples of systems or methods for manufacturing a sensor.

In some examples of this aspect, the solvent comprises propan-2-ol.

In some examples of this aspect, the compound of formula (I) comprises an equivalent weight between 800 and 1400.

In some examples of this aspect, the stoichiometry of the iridium oxide compound is IrOₓ; wherein x is equal to 2 or x is above 2.

The IrOX agent composition may be spray coated onto a working electrode and this working electrode may be suitable to determine pH.

A suitable system may comprise a spray coating head to deliver the IrOX agent composition on the working electrode. The working electrode may be arranged on an electrode substrate.

For reasons of completeness, various aspects of the present disclosure are set out in the following numbered clauses:
1. A method of additively manufacturing a sensor, the method comprising:
   providing an electrode, wherein the electrode comprises a conductive agent composition; and
   delivering a membrane agent composition onto the electrode to form a membrane.
2. The method according to clause 1, wherein the providing the electrode comprises:
   delivering the conductive agent composition onto an electrode substrate to form an electrode; and
   curing the conductive agent composition.
3. The method according to clause 2 comprising:
   heating the electrode substrate prior to the delivering the conductive agent composition onto the electrode substrate.
4. The method according to clause 3, wherein the electrode substrate is heated at a predefined temperature for a predetermined time.
5. The method according to clause 3 or 4, wherein the electrode substrate is a polymer.
6. The method according to clause 5, wherein the polymer is selected from at least one of the following: polyethylene terephthalate PET; polyethylene naphthalate PEN; polyimide such as Kapton; polycarbonate PC; poly(methyl methacrylate) PMMA; thermoplastic polyurethane TPU; and paper.
7. The method according to any of clauses 2 to 4 comprising:
   delivering a dielectric agent composition onto at least a portion of the electrode substrate prior to the delivering the conductive agent composition onto an electrode substrate.
8. The method according to clause 7, wherein the electrode substrate is a metal or a textile material.
9. The method according to clause 2 comprising:
   exposing the electrode substrate to oxygen plasma prior to the delivering the conductive agent composition onto an electrode substrate.
10. The method according to clause 9, wherein the electrode substrate is an oxide or a ceramic material.
11. The method according to any of clauses 2 to 10 comprising:
   screen-printing a conductive track onto the electrode substrate.
12. The method according to clause 11, wherein the conductive track comprises silver.
13. The method according to clause 11 or 12 comprising:
   curing the screen-printed conductive track by:
   heating the screen-printed conductive track; and
   exposing the screen-printed conductive track to an electromagnetic radiation with a wavelength comprised between 240 nm and 270 nm for 10 seconds.
14. The method according to any of clauses 1 to 13, wherein the conductive agent composition comprises a first conductive ink with at least one of silver; and silver chloride.
15. The method according to clause 14, wherein the electrode comprises a first reference electrode.
16. The method according to clause 14 or 15, wherein the curing the conductive agent composition comprises:
   curing the first conductive ink by heating the first conductive ink at 130°C for 30 minutes.
17. The method according to any of clauses 1 to 13, wherein the conductive agent composition comprises a second conductive ink with at least one of amorphous carbon; carbon black; and graphite.
18. The method according to clause 17, wherein the electrode comprises a counter electrode and / or a first working electrode and / or a second working electrode.
19. The method according to clause 17 or 18, wherein the curing the conductive agent composition comprises:
   curing the second conductive ink by heating the second conductive ink at 130°C for 30 minutes.
20. The method according to clause 18 or 19 comprising:
   spray coating a platinum agent composition onto the first working electrode.
21. The method according to clause 20 comprising:
   curing the platinum agent composition by heating the platinum agent composition at 80°C for 10 minutes.
22. The method according to clause 20 or 21, wherein the platinum agent composition is a carbon supported platinum composition with a 3%wt of platinum.
23. The method according to clauses 15 and 20 comprises obtaining a redox sensor and wherein the membrane agent composition is delivered onto the first reference electrode to form the membrane.
24. The method according to clause 23, wherein the formed membrane onto the electrode is an ion-saturated membrane.
25. The method according to clause 15, wherein the formed membrane onto the electrode is an ion-saturated membrane.
26. The method according to clause 18, wherein the formed membrane onto the electrode is an ion-selective membrane.
27. The method according to clauses 25 and 26 comprises obtaining a pH sensor.
28. A method of additively manufacturing a sensor assembly, comprising:
   providing a sensor onto an electrode substrate, wherein the sensor is manufactured using a method of additively manufacturing a sensor according to any of clauses 1 to 27;
   providing a first reference electrode on the electrode substrate according to clause 15; and
   spray coating an IrOX agent composition onto a second working electrode provided on the electrode substrate, wherein the provided first reference electrode and the IrOX spray coated second working electrode forms a pH sensor.
29. The method according to clause 28 comprising:
   curing the IrOX agent composition by heating the IrOX agent composition at 80°C for 10 minutes.
30. The method according to clause 28 or 29, wherein the IrOX agent composition comprises:
   an iridium oxide compound;
   a solvent; and
   a compound of formula (I): 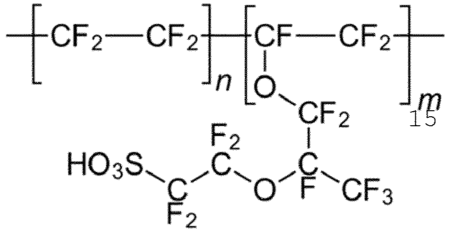 wherein n and m are integers which are above 1.
31. The method according to any of clauses 28 to 30 comprising:
   jetting a gold nanoparticle agent composition onto the second working electrode.
32. The method according to clause 31, wherein the gold nanoparticle agent composition comprises a gold particle with a size between 1 nm and 100 nm.
33. The method according to clauses 15 and 32 comprises obtaining a chlorine sensor.
34. The method according to any of clauses 1 to 13, wherein the conductive agent composition comprises a third conductive ink comprising silver.
35. The method according to clause 34, wherein the electrode comprises:
   a second reference electrode; and
   a third working electrode.
36. The method according to clause 35 comprises obtaining a conductivity sensor.
37. A method for manufacturing a microfluidic device comprising:
   providing a microfluidic device including a structure; wherein the structure comprises:
   an inlet channel;
   an outlet channel; and
   a detection chamber in fluid communication with the inlet channel and
   the outlet channel;
   connecting the microfluidic device with the sensor obtained by the method according to any of clauses 1 to 27, or with the sensor assembly according to any of clauses 28 to 36 such that the detection chamber is fluidically connected to the electrode of the sensor or to the electrode of the sensor assembly.
38. A system for additively manufacturing a sensor, the system comprising:
   a receiving region configured to receive an electrode;
   a membrane head for delivering a membrane agent composition onto the electrode through a nozzle, wherein the membrane head comprises a connecting port to receive an outlet end portion of a container to store the membrane agent composition;
   a container carrier configured to hold the container through a carrier end portion of the container; the container carrier being attached to the membrane head;
   a driving arm configured to drive the membrane head at least over the receiving region; and
   a membrane head actuator configured to feed the nozzle with the membrane agent composition, wherein the membrane head actuator is in fluid communication with the connecting port.
39. The system according to clause 38, wherein the container carrier comprises a carrier arm, and the carrier arm is connected to the membrane head through a carrier adapter.
40. The system according to clause 39, wherein the carrier arm is hingedly connected to the carrier adapter.
41. The system according to clause 39 or 40, wherein the carrier arm comprises a hollow portion configured to receive at least the carrier end portion of the container.
42. The system according to any of clauses 38 to 41, wherein the membrane head has a generally elongated configuration such that a membrane head longitudinal axis is defined, and the container being configured such that a container longitudinal axis is defined from the carrier end portion to the outlet end portion; wherein the container carrier is configured to hold the container in such a way that an angle is defined between the head longitudinal axis and the container longitudinal axis.
43. The system according to any of clauses 38 42, comprising:
   a container actuator configured to apply a pressure to the carrier end portion to deliver the membrane agent composition through the outlet end portion;
   wherein the system comprises a controller configured to control a synchronous operation of the container actuator and the membrane head actuator.
44. The system according to any of clauses 38 to 43, comprising:
   a conductive agent head for delivering a conductive agent composition onto an electrode substrate in the receiving region to form the electrode.
45. A sensor assembly comprising at least one of:
   a redox sensor according to the method of clause 23;
   a pH sensor according to the method of clause 27 or 28;
   a chlorine sensor according to the method of clause 33; and
   a conductivity sensor according to the method of clause 36.
46. A kit comprising:
   a sensor assembly according to clause 45; and
   a microfluidic device configured to be connected to the sensor assembly, the microfluidic device including a structure; wherein the structure comprises:
      an inlet channel;
      an outlet channel;
      a detection chamber in fluid communication with the inlet channel and
      the outlet channel; and
   such that the detection chamber is fluidically connected to at least an electrode of the sensor assembly.
47. The use of the kit according to clause 46:
   wherein a fluid sample is provided in the inlet channel;
   wherein the provided fluid sample is contacted with the electrodes or membranes;
   wherein the contact between the fluid sample and the electrodes or membranes generates a signal;
   wherein the signal is representative of:
      a presence of the target agent; and / or
      an oxidation-reduction potential of the fluid sample; and / or
      a pH of the fluid sample; and / or
      a temperature of the fluid sample; and / or
      a conductivity of the fluid sample.
48. An IrOX agent composition comprising:
   an iridium oxide compound;
   a solvent; and
   a compound of formula (I): 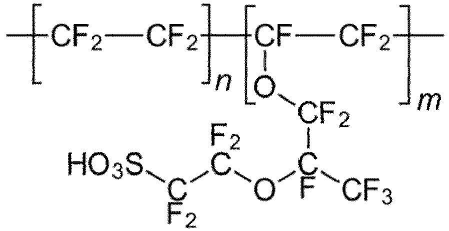 wherein n and m are integers which are above 1.
49. The IrOX agent composition of clause 47, wherein the solvent comprises propan-2-ol.
50. The IrOX agent composition of clause 48 or 49, wherein the compound of formula (I) comprises an equivalent weight between 800 and 1400.
51. The IrOX agent composition of any of clauses 48 to 50, wherein the stoichiometry of the iridium oxide compound is IrOₓ; wherein x is equal to 2 or x is above 2.
52. A method of additively manufacturing a pH sensor, the method comprising:
   providing an electrode, wherein the electrode comprises a conductive agent composition; and
   spray coating an IrOX agent composition onto the electrode.
53. The method according to clause 52, wherein the providing the electrode comprises:
   delivering the conductive agent composition onto an electrode substrate to form an electrode; and
   curing the IrOX agent composition.
54. The method according to clause 53 comprising:
   curing the IrOX agent composition by heating the IrOX agent composition at 80°C.
55. The method according to any of clauses 52 to 54, wherein the IrOX agent composition comprises:
   an iridium oxide compound;
   a solvent; and
   a compound of formula (I): 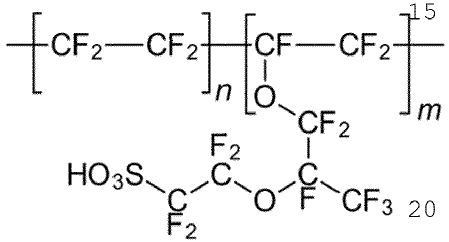 wherein n and m are integers which are above 1.
56. The method according to any of clauses 52 to 55, wherein the conductive agent composition comprises a first conductive ink with at least one of silver; and silver chloride.
57. The method according to clause 56, wherein the electrode comprises a first reference electrode.
58. The method according to any of clauses 52 to 55, wherein the conductive agent composition comprises a second conductive ink with at least one of amorphous carbon; carbon black; and graphite.
59. The method according to clause 58, wherein the curing the conductive agent composition comprises:
   curing the second conductive ink by heating the second conductive ink at 130°C for 30 minutes.
60. The method according to clause 58 or 59, wherein the electrode comprises a counter electrode and / or a second working electrode.
61. The method according to clause 60, wherein the IrOX agent composition is spray coated onto the second working electrode.
62. A method of additively manufacturing a chlorine sensor, the method comprising:
   providing an electrode, wherein the electrode comprises a conductive agent composition; and
   jetting a gold nanoparticle composition onto the electrode.
63. The method according to clause 62 wherein the providing the electrode comprises:
   delivering the conductive agent composition onto an electrode substrate to form an electrode; and
   curing the gold nanoparticle composition.
64. The method according to clause 62 or 63, wherein the conductive agent composition comprises a first conductive ink with at least one of silver; and silver chloride.
65. The method according to clause 64, wherein the electrode comprises a first reference electrode.
66. The method according to clause 62 or 63, wherein the conductive agent composition comprises a second conductive ink with at least one of amorphous carbon; carbon black; and graphite.
67. The method according to clause 66, wherein the curing the conductive agent composition comprises:
   curing the second conductive ink by heating the second conductive ink at 130°C for 30 minutes.
68. The method according to clause 66 or 67, wherein the electrode comprises a counter electrode and / or a second working electrode.
69. The method according to any of clauses 62 to 68, wherein the gold nanoparticle composition comprises a gold particle with a size between 1 nm and 100 nm.
70. A system for additively manufacturing a sensor, the system comprising:
   a first delivery unit configured to deliver a conductive agent composition onto an electrode substrate to form an electrode;
   a curing unit to cure the delivered conductive agent composition;
   a second delivery unit configured to:
      providing an electrode, wherein the electrode comprises a conductive agent composition; and
      delivering a membrane agent composition onto the electrode to form a membrane .
71. The system according to clause 70, wherein the secondary delivery unit comprises:
   a receiving region configured to receive the electrode;
   a membrane head for delivering a membrane agent composition onto the electrode through a nozzle, wherein the membrane head has a connecting port to receive an outlet end portion of a container to store the membrane agent composition;
   a container carrier configured to hold the container through a carrier end portion of the container; the container carrier being attached to the membrane head;
   a driving arm configured to drive the membrane head at least over the receiving region; and
   a membrane head actuator configured to feed the nozzle with the membrane agent composition, wherein the membrane head actuator is in fluid communication with the connecting port
72. The system according to clause 70 or 71 comprising:
   an illuminating device to emit an electromagnetic radiation with a wavelength comprised between 100 nm and 400 nm on the electrode substrate.

Although only a number of examples have been disclosed herein, other alternatives, modifications, uses, and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. A method of additively manufacturing a sensor, the method comprising:
providing an electrode, wherein the electrode comprises a conductive agent composition; and
delivering a membrane agent composition onto the electrode to form a membrane.

2. The method according to claim 1, wherein the conductive agent composition comprises at least one of:
a first conductive ink with at least one of silver; and silver chloride; and
a second conductive ink with at least one of amorphous carbon; carbon black; and graphite.

3. The method according to claim 2, wherein the electrode comprises a first reference electrode made of the first conductive ink.

4. The method according to claim 2, wherein the electrode comprises a first working electrode made of the second conductive ink.

5. The method according to claim 4 comprising:
spray coating a platinum agent composition onto the first working electrode.

6. The method according to claims 3 and 5, wherein the sensor is a redox sensor and wherein the membrane agent composition is delivered onto the first reference electrode to form the membrane.

7. The method according to claim 3, wherein the formed membrane onto the electrode is an ion-saturated membrane.

8. The method according to claim 4, wherein the formed membrane onto the electrode is an ion-selective membrane.

9. The method according to claim 7 and 8, wherein the sensor comprises a pH sensor.

10. A method of additively manufacturing a sensor assembly, comprising:
providing a sensor onto an electrode substrate, wherein the sensor is manufactured using a method of additively manufacturing a sensor according to any of claims 1 to 9;
providing a first reference electrode on the electrode substrate according to claim 3; and
spray coating an IrOX agent composition onto a second working electrode provided on the electrode substrate, wherein the provided first reference electrode and the IrOX spray coated second working electrode forms a pH sensor.

11. The method according to claim 10, wherein the IrOX agent composition comprises:
an iridium oxide compound;
a solvent; and
a compound of formula (I): 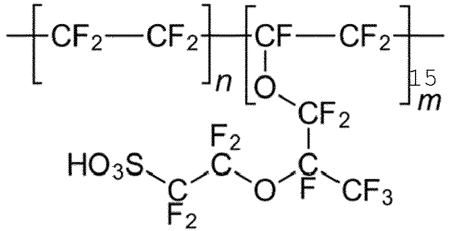 wherein n and m are integers which are above 1.

12. The method according to claim 10 comprising:
jetting a gold nanoparticle agent composition onto the second working electrode.

13. The method according to claim 12, wherein the sensor assembly comprises a chlorine sensor.

14. A method for manufacturing a microfluidic device comprising:
providing a microfluidic device including a structure; wherein the structure comprises:
an inlet channel;
an outlet channel; and
a detection chamber in fluid communication with the inlet channel and
the outlet channel;
connecting the microfluidic device with the sensor obtained by the method according to any of claims 1 to 9, or with the sensor assembly according to any of claims 10 to 13 such that the detection chamber is fluidically connected to the electrode of the sensor or of the sensor assembly.

15. A system for additively manufacturing a sensor, the system comprising:
a receiving region configured to receive an electrode;
a membrane head for delivering a membrane agent composition onto the electrode through a nozzle, wherein the membrane head comprises a connecting port to receive an outlet end portion of a container to store the membrane agent composition;
a container carrier configured to hold the container through a carrier end portion of the container; the container carrier being attached to the membrane head;
a driving arm configured to drive the membrane head at least over the receiving region; and
a membrane head actuator configured to feed the nozzle with the membrane agent composition, wherein the membrane head actuator is in fluid communication with the connecting port.
